# EUROPEAN PATENT SPECIFICATION

(11) **EP 2 514 378 B1**
(45) Date of publication and mention of the grant of the patent: **18.02.2015**
(21) Application number: 12176780.0
(22) Date of filing: 10.12.2010
(51) Int. Cl.: A61B 17/70

(54) **Bone anchoring device**
Knochenverankerungsvorrichtung
Dispositif d'ancrage d'os

(43) Date of publication of application: 24.10.2012
(62) Divisional of application: 10194601.0
(73) Proprietor: Biedermann Technologies GmbH & Co. KG, 78166 Donaueschingen (DE)
(72) Inventor: Biedermann, Lutz, 78048 VS-Villingen (DE); Matthis, Wilfried, 79367 Weisweil (DE); Dannecker, Berthold, 78112 St. Georgen (DE)
(74) Representative: Prüfer & Partner GbR European Patent Attorneys

(56) References cited:
- EP-A1- 2 022 423
- EP-A1- 2 201 903
- WO-A2-2007/038350

## Description

The invention relates to a bone anchoring device comprising a bone anchoring element and a receiving part for connecting the bone anchoring element to a rod. The head of the bone anchoring element is locked in the receiving part by compression of a portion of the receiving part laterally surrounding the head by means of a locking ring, which is pressed down by the rod. The locking ring has two upwardly extending projections that extend into the channel for the rod and can be engaged by the rod to exert pressure onto the locking ring. The upwardly extending projections cooperate with the receiving part in such a way that the locking ring can assume a position in which the head is pivotable in the head receiving portion but can not be removed from the head receiving portion.

A bone anchoring device, which includes a bone anchoring element and a receiving part with a rod receiving portion and a head receiving portion and a locking ring for locking the head in the head receiving portion is known from EP 2 022 423 A1. Below the bottom of the channel for the rod in the rod receiving portion cutouts are provided on either end of the channel, through which two upwardly extending projections of the locking ring extend into the channel, so that they are engageable by the rod. In one embodiment, the upwardly extending projections have lateral projections, which snap into the channel to prevent the locking ring from falling off when the head is not inserted into the head receiving portion.

WO 2007/038350 A2 describes an apparatus for connecting a bone anchor to a support rod, the apparatus including a connector body and a cap. The connector body has a channel to receive and locate the support rod relative to the bone anchor. A sleeve fits over a socket portion of the connector body in a temporary position, in which the sleeve permits insertion of the bone anchor into the socket. The sleeve can then be moved to a provisional locking position, in which the sleeve permits angulation but prevents removal of the bone anchor and can further be moved to a locking position in which the sleeve prevents both angulation and removal of the bone anchor.

WO 2007/038350 A2 discloses an apparatus for connecting a bone anchor to a support rod, comprising a connector body having a top opening, two opposed arms and a channel to receive and locate the support rod relative to the bone anchor, the channel communicating with the top opening, and a cap to secure the support rod in the channel, the cap having at least one radial projection and being configured to move axially into the top opening of the connector body into a partially installed position covering the top opening, and configured to rotate to an installed position without moving axially relative to the connector body.

It is the object of the invention to provide an improved bone anchoring device, which has a small size and which allows a safe handling during surgery.

The object is solved by a bone anchoring device according to claim 1. Further developments are given in the dependent claims.

The bone anchoring device provides a pre-locking position for the locking ring, in which the head of the screw element can be pivoted but cannot be removed from the head receiving portion. The pre-locking position is achieved by an engagement of the locking ring with the receiving part in the region of the rod supporting projections, which extend into the channel for the rod. This allows to simplify the design of the locking ring in the area, which clamps the head. Also, the overall size, in particular with respect to the largest outer diameter of the bone anchoring device, can be decreased. Therefore, the bone anchoring device is particularly suitable for applications in the cervical spine.

The pre-locking position of the locking ring guarantees safe handling during surgery, since the head of the bone anchoring element cannot be removed from the receiving part inadvertently.

Further features and advantages of the invention will become apparent from the description of embodiments using the accompanying drawings.

In the drawings:
- Fig. 1:: shows a perspective exploded view of an embodiment of the bone anchoring device.
- Fig. 2:: shows a perspective view of the bone anchoring device of Fig. 1 in an assembled state.
- Fig. 3a):: shows a cross-sectional view of the bone anchoring device of Fig. 2, the section being taken perpendicular to the rod axis.
- Fig. 3b):: shows an enlarged portion of Fig. 3a).
- Fig. 4:: shows a perspective view of a locking ring of the bone anchoring device.
- Fig. 5:: shows a side view of the locking ring of Fig. 4 in a first condition.
- Fig. 6:: shows a side view of the locking ring of Fig. 4 in a second condition.
- Fig. 7:: shows a side view of the receiving part and the locking ring of Fig. 1, wherein the locking ring is mounted from the bottom end of the receiving part.
- Fig. 8a):: shows a side view of the receiving part with mounted locking ring during a step of entering the first position of the locking ring, which allows the introduction of the head of the bone anchoring element.
- Fig. 8b):: shows an enlarged portion of Fig. 8a).
- Fig. 9a):: shows a perspective view of the receiving part with locking ring mounted thereon in the first position, which allows the introduction of the head of the bone anchoring element.
- Fig. 9b):: shows an enlarged portion of Fig. 9a).
- Fig. 10a):: shows a side view the bone anchoring device of Fig. 1 with the head of the anchoring element introduced into the receiving part and the locking ring in a second position.
- Fig. 10b):: shows an enlarged portion of Fig. 10a).
- Fig. 11a):: shows a side view of the bone anchoring device with an inserted rod and a locking ring in a third position, in which it locks the head in the receiving part.
- Fig. 11b):: shows an enlarged portion of Fig. 11a).

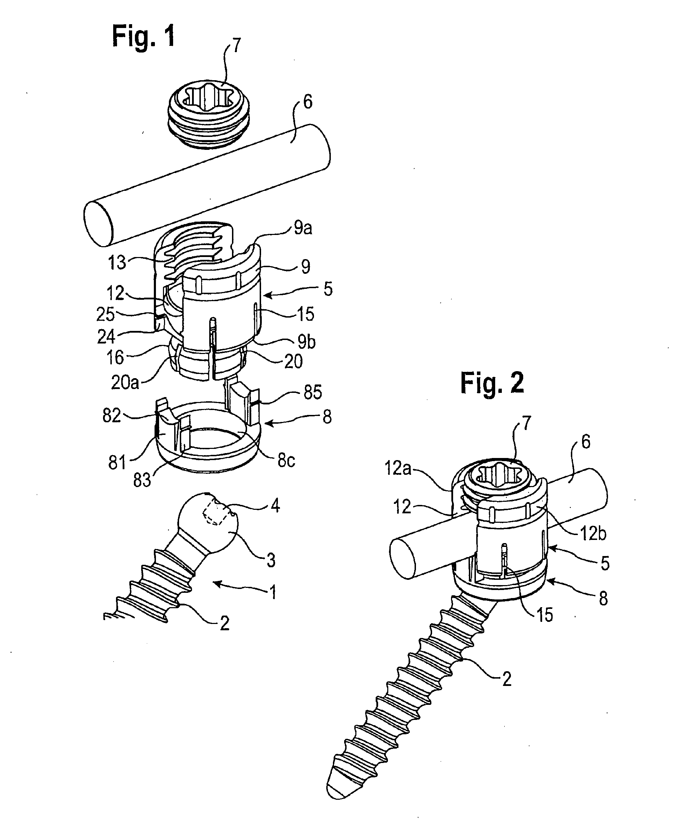

As shown in Figs. 1 to 3b), the bone anchoring device according to an embodiment comprises a bone anchoring element 1 in the form of a bone screw having a threaded shaft 2 and a spherical segment-shaped head 3. The head 3 has a recess 4 for engagement with a driver. The bone anchoring device further includes a receiving part 5 for receiving a rod 6 to connect it to the bone anchoring element 1. Further, a closure element 7 in the form of an inner screw or set screw is provided for securing the rod 6 in the receiving part 5. In addition, the bone anchoring device includes a locking ring 8 for locking the head 3 in the receiving part 5.

The receiving part 5 comprises a rod receiving portion 9, which has a first end 9a and an opposite second end 9b. The rod-receiving portion 9 is substantially cylindrical and comprises a coaxial first bore 10 provided at the second end 9b. The diameter of the first bore 10 is smaller than the diameter of the head 3 of the bone anchoring element 1. Therefore, the head 3 cannot extend into the rod receiving portion 9. The rod receiving portion 9 further comprises a coaxial second bore 11 extending from the first end 9a to a distance from the second end 9b. The diameter of the second bore 11 is larger than that of the first bore 10 and larger than the diameter of the rod 6. A U-shaped recess 12 is provided in the rod receiving portion 9, which extends from the first end 9a to the second end 9b. The U-shaped recess 12 forms a channel for the rod which has a size such that the rod 6 can be placed and guided therein. By means of the U-shaped recess 12 two free legs 12a, 12b are formed, on which an internal thread 13 is provided. The internal thread can be a metric thread, a flat thread, a negative angle thread, a saw-tooth thread or any other thread. Preferably, a thread form such a flat thread or a negative angle thread is used, which prevents splaying of the legs 12a, 12b when the inner screw 7 is screwed-in. The depth of the U-shaped recess 12 is such that the rod 6 and the inner screw 7 can be inserted between the legs 12a, 12b. Between the bottom of the recess 12, a substantially flat section 14 is provided forming the end of the bore 11.

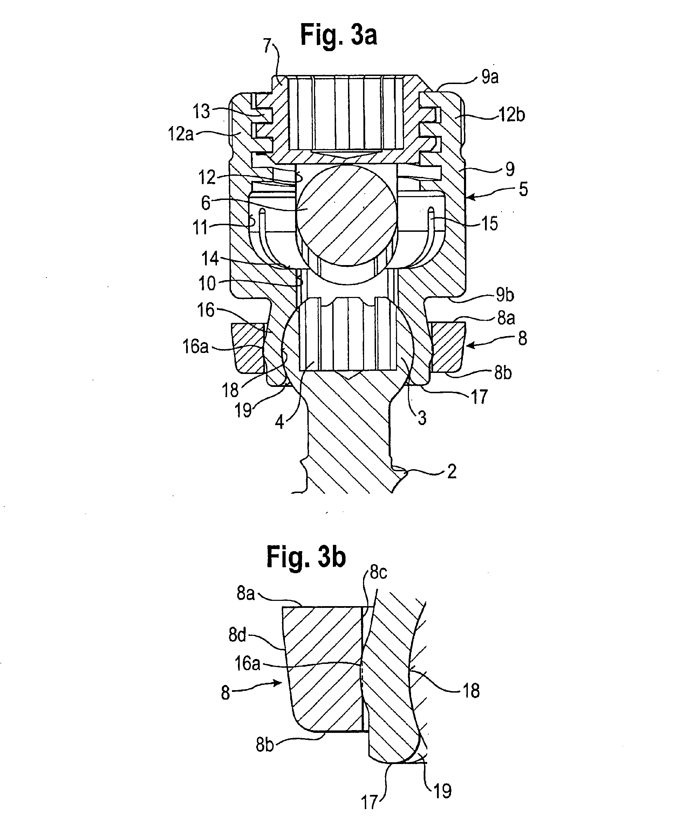

The rod receiving portion 9 of the receiving part 5 has a plurality of coaxial slits 15 extending from the second end 9b to a distance from the first end 9a, the distance corresponding approximately to the length of the internal thread 13. The slits are open at the second end 9b and extend through the substantially flat section 14, as can be seen for example in Fig. 3a). The number of slits is provided according to the desired degree of elasticity to facilitate introduction of the head 3 into the receiving part. It may depend on the material and the wall thickness and/or other factors.

As can be seen in particular in Figs. 1 and 7 to 9, the rod receiving portion 9 has cutouts 24, which extend from the second end 9b into the bottom of the U-shaped recess 12. The cutouts 24 are provided at either end of the U-shaped recess 12 seen in a direction along the channel axis. At the transition of the cutouts 24 to the vertical wall of the U-shaped recess 12 inwardly extending noses 25 ar formed, each nose having a vertical inner wall portion 25a and perpendicular thereto a horizontal lower wall portion 25b. The noses 25 are provided on either side of the channel formed by the U-shaped recess so that each cutout 24 is provided with two noses 25, respectively. By the noses 25 the width of the cutouts 24 is reduced.

Adjacent to the second end 9b, the receiving part 5 includes a head receiving portion 16 providing an accommodation space for the head 3 of the bone anchoring element 1. The head receiving portion 16 has a free end 17 opposite to the second end 9b of the rod receiving portion 9. As can be seen in particular in Fig. 3 a), a maximum outer diameter of the head receiving portion 16 is smaller than the outer diameter of the rod receiving portion 9 so that the head receiving portion 16 is recessed with respect to the rod receiving portion 9. The head receiving portion 16 has an internal hollow section 18, which is in the embodiment shown spherically-shaped with a radius matching the radius of the head 3 of the bone anchoring element. The internal hollow section 18 has an opening 19 at the free end 17. The dimension of the internal hollow section 18 is such that the hollow section 18 encompasses the head 3 of the bone anchoring element including a region of the largest diameter of the head 3.

As can be seen in particular in Fig. 1, slits 20 are provided in the head receiving portion 16 which are open to the free end 17 and continue in the slits 15 of the rod receiving portion. There may also be slits 20a, which end in the head receiving portion at a position above the largest diameter of the head. For example, slits 20a, which are at positions of the U-shaped recess, may be provided. The slits in the head receiving portion 16 render the head receiving portion 16 flexible, such that the head 3 of the anchoring element can be inserted by expanding the head receiving portion and can be clamped by compression the head receiving portion.

The outer surface of the head receiving portion 16 comprises an outwardly curved section 16a, which serves for a cooperation with the locking ring 8 described below.

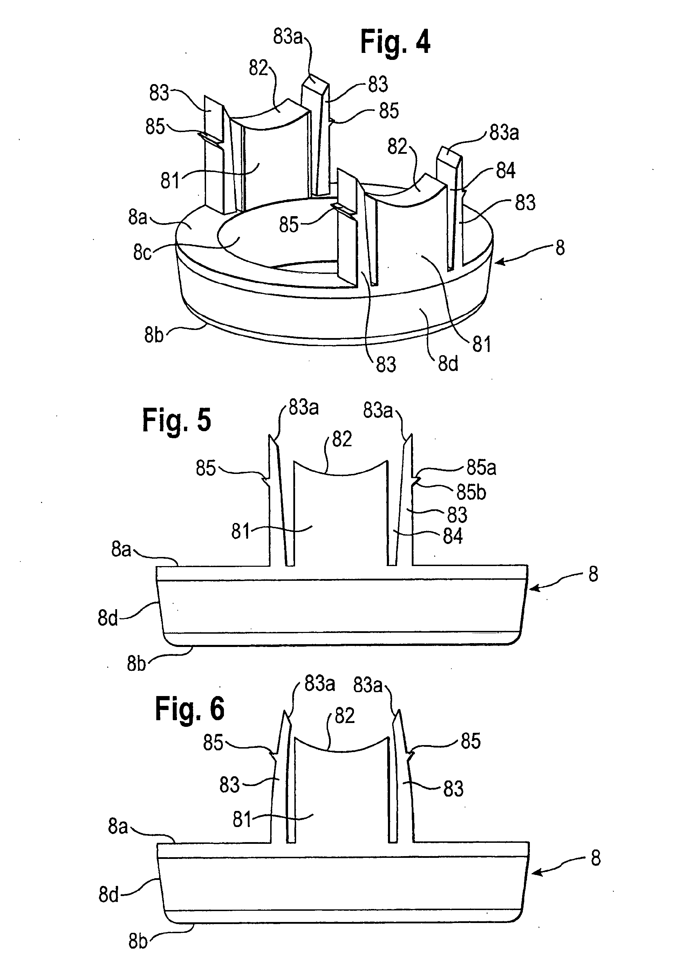

The locking ring 8 will now be described with reference to Figs. 4 to 6. The locking ring 8 has an upper end 8a facing the second end 9b of the rod receiving portion 9 of the receiving part in a mounted state and a lower end 8b. The inner wall of the locking ring, has a portion 8c that is substantially cylindrical. The portion 8c may extend from the upper end 8a to the lower end 8b but is at least provided in a region cooperating with the curved outer surface portion 16a of the head receiving portion 16. The inner diameter of the cylindrical portion 8c is slightly smaller than the outer diameter of the curved portion 16a of the head receiving portion to allow a compression of the head receiving portion by the locking ring 8. The flexibility of the head receiving portion 16 allows to mount the locking ring from the free end 17 onto the head receiving portion 16 by slightly compressing the head receiving portion 16. The locking ring 8 further has a substantially conical or cylindrical outer surface 8d with an outer diameter that is the same or smaller than the outer diameter of the rod receiving portion 9, so that, as shown in Fig. 2, when the locking ring is mounted on the receiving part, it does not extend radially outwards from the receiving part 5. This provides for a compact shape of the bone anchoring device.

The height of the locking ring 8 in an axial direction is smaller than that of the head receiving portion 16 of the receiving part 5 so that, as shown in Fig. 3a), there is a distance between the upper side of the locking ring 8 and the second end 9b of the rod receiving portion 9, when the locking ring is in a position around the head 3.

The locking ring 8 further comprises at its upper end 8a two projections 81 located diametrically opposite to each other. The projections 81 have such a height that they extend into the cutouts 24 and project above the bottom of the U-shaped recess 12 when the locking ring is in a position, in which the head 3 is not locked. The free end 82 of the projection 81 can be curved with a curvature corresponding to the curvature of the rod 6.

At either side of each projection 81, an upwardly extending spring portion 83 is provided. The spring portions 83 are flexible in such a way that they can be elastically bent towards the projections 81. The cross-section of the spring portions 83 is substantially trapezoidal with the thickness decreasing towards the free end 83a. The free end 83a can be adapted to the shape of the rod 6, so that the rod 6, when it is introduced into the U-shaped recess, rests on the upper surface 82 of the projections 81 and on the free end 83a of the spring portions 83. The flexibility of the spring portions 83 depends on the thickness of the spring portions in circumferential direction and on the size of the gap 84 between the projection 81 and the spring portions 83. Each spring portion 83 comprises at a distance from the upper end 8a a horizontally outward extending projection 85 with a horizontal upper side 85a and an inclined lower side 85b. The horizontally extending projections 85 are positioned at such a height that when they cooperate with the noses 25 as explained below, the upper surface 82 of the projections 81 extends into the U-shaped recess 12.

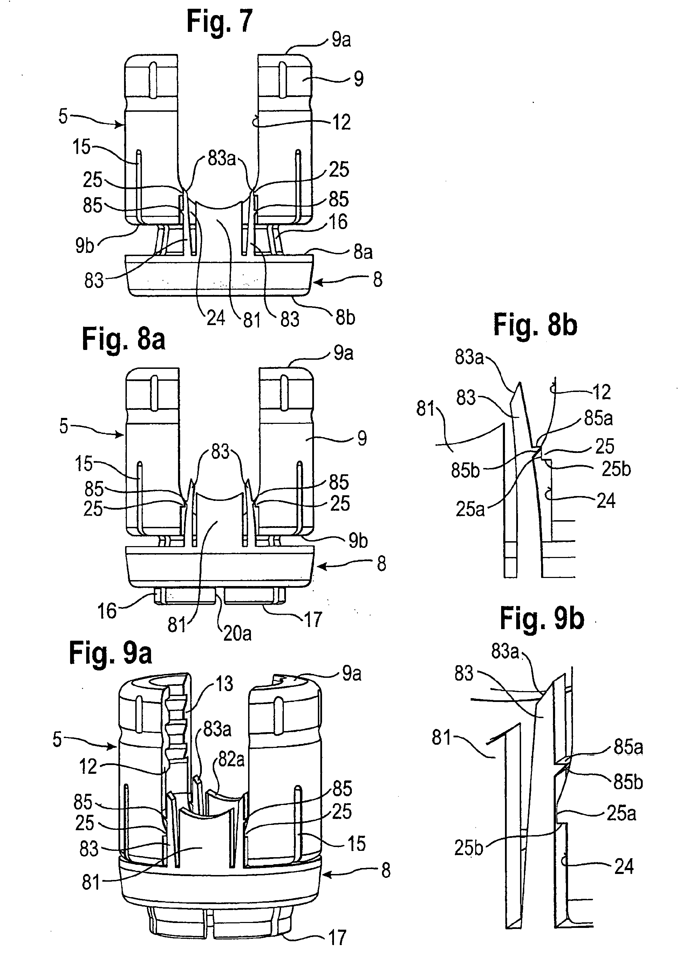

When the upwardly extending spring portions 83 are not deflected, they extend through the cutouts 24 as shown in Fig. 7. The distance of the outermost edge of the horizontally extending projections 85 is larger than the distance between the vertical inner walls 25a of the two noses 25 as also shown in Fig. 7. By deflecting the spring portions 83, the distance between the outer edge of the horizontally extending projections 85 is decreased, as shown in Fig. 8a).

The inner screw 7 has a thread cooperating with the internal thread 13 provided on the legs 12a, 12b. If a thread form which prevents the legs from splaying is used, a single closure element such as the inner screw 7 is sufficient. This reduces the size of the bone anchoring device in a radial direction.

The receiving part, the locking ring, the inner screw and the bone anchoring element are made of a bio-compatible material, for example of titanium or stainless steel or a bio-compatible alloy, such as Nitinol or of a bio-compatible plastic material such as, for example, polyether-etherketone (PEEK). The parts may be all of the same material or of different materials.

The steps of mounting the locking ring onto the receiving part will be described with reference to Figs. 7 to 9b). In a first step, as shown in Fig. 7, the locking ring is mounted from the free end 17 of the head receiving portion such that the projections 81 are aligned with the U-shaped recess 12. The projections 81 and the upwardly extending spring portions 83 are introduced into the cutouts 24 until the upper edge 83a is above the nose 25. The horizontally extending projections 85 are still within the cutouts 24.

Then, as shown in Figs. 8a) and 8b), the upwardly extending spring portions 83 are deflected towards the projection 81 manually or with an instrument, so that the horizontally extending projections 85 snap behind the noses 25 into the U-shaped recess 12. This condition is shown in Figs. 9a) and 9b). When the horizontal projections 25 are above the noses, the upwardly extending spring portions 83 can expand and assume the undeflected position again. In this position, the locking ring 8 is secured against falling off, since it is loosely held by the noses 25. Furthermore, the locking ring is secured against rotation, since the spring portions 83 and the projections 81 extend into the U-shaped recess 12. The locking ring 8 can be shifted upwards until its upper edge 8a abuts against the second end 9b as shown in Figs. 9a) and 9b). The cylindrical inner wall portion 8c of the locking ring is above the curved outer surface portion 16a of the head receiving portion 16, so that the head receiving portion 16 is not compressed. Between the cylindrical inner wall portion 8c of the locking ring 8 and the outer surface of the head receiving portion, there is a space which allows an expansion of the head receiving portion 16 for the introduction of the head 3 of the bone anchoring element 1. The receiving part 5 and the locking ring may be pre-assembled in such a way that the locking ring is in the open position as shown in Figs. 9a) and 9b).

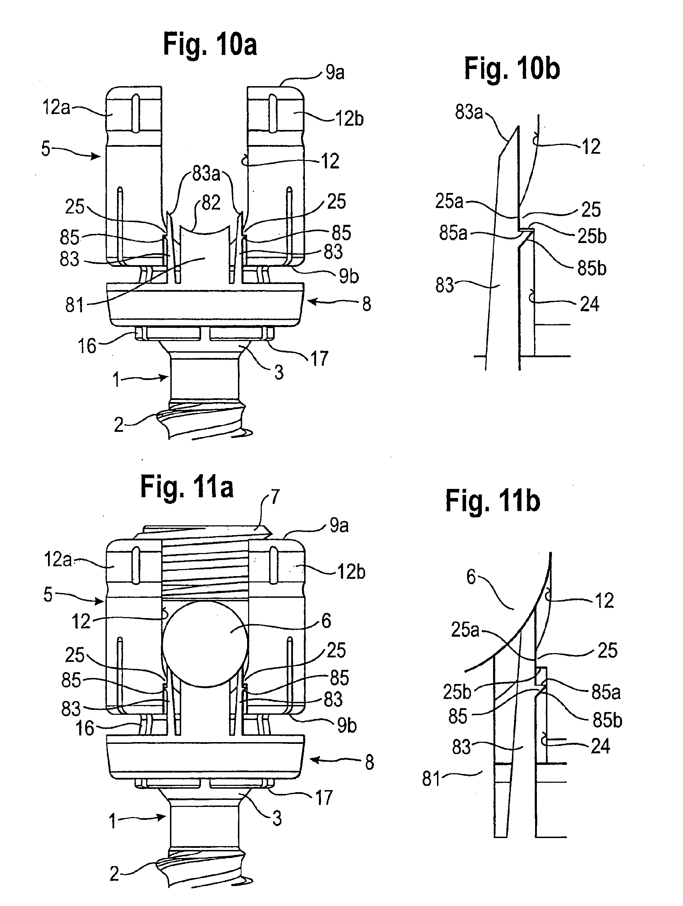

The assembly and function of the bone anchoring device will now be described with reference to Figs. 10a) to 11b). After the introduction of the head 3 from the free end 17 into the internal hollow section 18 of the head receiving portion, the locking ring 8 is shifted downward towards the free end 17 manually or with an instrument. During this step, the inclined lower surface 85b of the horizontally extending projections 85 slides along the curved portion of the U-shaped recess 12. Since the width of the U-shaped recess gradually decreases in the curved portion, the upwardly extending spring portions 83 are deflected towards the projections 81 until the horizontally extending projections 85 snap under the noses 25. The cutouts 24 provide a space for the horizontally extending projections 85 so that the upwardly extending spring portions 83 can assume their undeflected state. In the position shown in Figs. 10a) and 10b), the locking ring is prevented from moving upward by the abutment of the upper horizontal surface 85a to the lower horizontal surface 25b of the nose 25 forming a stop. Since the locking ring is in a position, which at least partly covers the curved outer surface portion 16a of the head receiving portion, the head receiving portion 16 is no longer expandable, which prevents removal of the head 3 from the head receiving portion 16. This position of the locking ring is the pre-locking position. If the size of the locking ring and the head receiving portion is such that in the pre-locking position the head receiving portion is slightly compressed, the bone anchoring element may be held in a preliminary angular position with respect to the receiving part by friction. The bone anchoring element is still pivotable by applying a force greater than the friction force to pivot the bone anchoring element.

In Figs. 11a) and 11b) the locking ring assumes a third position with respect to the receiving part, in which it is further shifted downward by the rod 6, which presses onto the upper surface 82 of the projection 81 and onto the upper portion 83a of the upwardly extending spring portions 83, when the inner screw 7 is tightened. The third position is the locking position. When shifting downward, the locking ring 8 exerts an increasing compression force onto the head receiving portion which finally locks the head 3 in a desired angular position within the head receiving portion 16.

There are different ways of clinical use of the bone anchoring device. If the locking ring and the receiving part are pre-assembled, it is possible to select a suitable bone anchoring element and connect it to the receiving part, when the locking ring is in the first position shown in Figs. 9a) and 9b). This can be done at any time before or during surgery. The bone anchoring device can in this case be provided as a modular system, in which the surgeon can select a suitable bone anchoring element to be connected to the receiving part.

Alternatively, the receiving part, the locking ring and the bone anchoring element can be pre-assembled and provided in the pre-locking condition. Usually, several bone anchoring devices are to be connected to a spinal rod 6. In a first step, the bone anchoring elements are inserted into the bone parts or vertebrae by engaging the recess 4 in the head 3 with a driver. It may happen that the free end 17 of the head receiving portion abuts against the bone surface. In this case, the receiving part and the bone anchoring element are not disassembled, since in the pre-locking position as shown in Fig. 10a) and 10b), the head 3 is prevented from removal from the head receiving portion 16.

After inserting the bone anchoring element in the bone, the receiving parts are adjusted by pivoting them with respect to the bone anchoring elements to be aligned with the rod to be inserted. After insertion of the rod, the inner screws are tightened to press the rod against the upper surface 82 and 83a of the projections and the spring portions, which moves the locking ring downward to compress the head receiving portion to lock the head therein.

Although, the bone anchoring device is shown to have two upwardly extending spring portions, which are arranged on either side of the projection 81, only one spring portion is sufficient. The spring portions and the noses are not limited to the specific shape shown in the embodiment, other shapes are possible, which fulfil the function that the locking ring is held by a stop in a pre-locking position. For example, a spring portion could be provided at the inner wall of the U-shaped recess, which engages into a rigid portion provided at the locking ring. Also, the projections 81 can be omitted and their function can be fulfilled by the upwardly extending spring portion 83.

For the anchoring element, all kinds of anchoring elements can be used and combined with the receiving part. These anchoring elements are e.g. screws of different length, with different diameters, cannulated screws, screws with different thread forms, nails, etc. The head of the screw and the shaft can also be separate parts, which are connectable to each other.

The shape of the receiving part is not limited to the embodiment shown. For example, the slits in the rod receiving portion can be omitted. Further, the receiving part can have an asymmetric or inclined end portion for allowing a greater pivot angle of the screw member to one side.

The channel for the rod can be closed on the first end of the rod receiving portion. For the closure element other types such as outer nuts or other closure elements can be used.

## Claims

1. Bone anchoring device including
a bone anchoring element (1) having a shaft (2) for anchoring in the bone and a head (3);
a receiving part (5) for coupling a rod (6) to the bone anchoring element, wherein the receiving part comprises
a rod receiving portion (9) with a first end (9a) and a second end (9b) and a channel (12) with a bottom for receiving the rod, and
a head receiving portion (16) at the side of the second end (9b) for accommodating the head (3), the head receiving portion having a free end (17) and being flexible so as to allow introduction and clamping of the head;
a locking ring (8) embracing the head receiving portion (16);
wherein the head (3) is locked by compressing the head receiving portion (16) with the locking ring;
a first engagement portion (85) is provided at the locking ring and a second engagement portion (25) is provided at the inner wall of the channel (12);
**characterized in that**
the first engagement portion (85) is provided on a spring portion (83) that extends into the channel (12) and that the first engagement portion (85) engages the second engagement portion (25) to hold the locking ring (8) in a position in which the head (3) is pivotable in the head receiving portion and is prevented from removal therefrom.

2. The bone anchoring device claim 1, wherein the first engagement portion (85) has a surface (85a) that abuts against a surface (25a) of the second engagement portion (25) when the locking ring is moved towards the second end (9b).

3. The bone anchoring device of claim 1 or 2, wherein the first engagement portion (85) is configured to flexibly move with respect to the second engagement portion (25).

4. The polyaxial bone anchoring device according to one of claims 1 to 3, wherein the rod receiving portion (9) comprises at least one cutout (24) which extends at one end of the channel from the bottom to the second end (9b) and wherein the locking ring has at least one projection (81, 83) which extends through the cutout (24) into the channel (12) when the locking ring is in a position in which the head is not locked.

5. The bone anchoring device of claim 4, wherein the projection (81) and the spring portion (81, 83) are separated and the spring portion (83) the can be flexibly moved towards the projection (81).

6. The bone anchoring device of one of claims 1 to 5, wherein the free end (83a) of the spring portion (83) provides a support for the rod (6).

7. The bone anchoring device of one of claims 4 to 6, wherein cutouts (24) are provided at either end of the channel and two diametrically opposite projections (81) are provided at the locking ring that extend through the cutouts (24) into the channel and wherein corresponding to each projection (81) at least one engagement portion (85) is provided.

8. The bone anchoring device of one of claims 1 to 7, wherein the locking ring (8) can assume an open position in which the head receiving portion (16) is not compressed to allow introduction of the head (3), and wherein preferably in the open position the first engagement portion (85) extends into the channel (12).

9. The bone anchoring device of one of claims 1 to 8, wherein the locking ring (8) can assume a locking position in which it compresses the head receiving portion (16) to lock the head.

10. The bone anchoring device of claim 9, wherein in the locking position the first engagement portion (85) extends into the cutout (24) and is out of engagement with the second engagement portion (25).

11. The bone anchoring device of one of claims 1 to 10, wherein the first and the second engagement portions extend substantially in a circumferential direction of the locking ring (8).

12. The bone anchoring device according to one of claims 1 to 11, wherein the diameter of the head receiving portion (16) of the receiving part (5) adjacent to the second end is smaller than the diameter of the rod receiving portion (9) at the second end (9b), and/or wherein the outer diameter of the locking ring (8) is smaller than or equal to the outer diameter of the rod receiving portion (9).

13. The bone anchoring device according to one of claims 1 to 12, wherein the head receiving portion (16) comprises a plurality of slits (20) being open to the free end (17) and/or wherein the rod receiving portion (9) comprises a plurality of slits (15) in its wall.

14. The bone anchoring device according to one of claims 1 to 13, wherein the head (3) is spherical segment-shaped and the second portion comprises an inner surface with a corresponding spherical portion (18) to allow a pivotal movement of the head (3).

15. The bone anchoring device according to one of claims 1 to 14, wherein a closure element (7), preferably a set screw, is provided for securing the rod in the recess.

## Patentansprüche

1. Knochenverankerungsvorrichtung, umfassend:
ein Knochenverankerungselement (1) mit einem Schaft, zum Verankern in dem Knochen, und einen Kopf (3);
ein Aufnahmeteil (5) zum Verbinden eines Stabs (6) mit dem Knochenverankerungselement, wobei das Aufnahmeteil umfasst:
einen Stabaufnahmeabschnitt (9) mit einem ersten Ende (9a), einem zweiten Ende (9b) und einem Kanal (12) mit einem Boden zum Aufnehmen des Stabs, und
einen Kopfaufnahmeabschnitt (16) auf der Seite des zweiten Endes (9b) zum Aufnehmen des Kopfs (3), wobei der Kopfaufnahmeabschnitt ein freies Ende (17) besitzt und flexibel ist, so dass das Einführen und Klemmen des Kopfs ermöglicht ist;
einen Klemmring (8), welcher den Kopfaufnahmeabschnitt (16) umschließt;
wobei der Kopf (3) durch ein Zusammendrücken des Kopfaufnahmeabschnitts (16) anhand des Klemmrings geklemmt wird;
einen ersten Eingriffsabschnitt (85), der an dem Klemmring vorgesehen ist, und einen zweiten Eingriffsabschnitt (25), der an der Innenwand des Kanals (12) vorgesehen ist;
**dadurch gekennzeichnet, dass**
der erste Eingriffsabschnitt (85) auf einem Federabschnitt (83) vorgesehen ist, der sich in den Kanal (12) erstreckt, und dass der erste Eingriffsabschnitt (85) mit dem zweiten Eingriffsabschnitt (25) in Eingriff tritt, um den Klemmring (8) in einer Position zu halten, in welcher der Kopf (3) in dem Kopfaufnahmeabschnitt schwenkbar ist, und in welcher der Kopf (3) an einer Entfernung aus dem Kopfaufnahmeabschnitt gehindert ist.

2. Die Knochenverankerungsvorrichtung gemäß Anspruch 1, wobei der erste Eingriffsabschnitt (85) eine Oberfläche (85a) besitzt, die an eine Oberfläche (25a) des zweiten Eingriffsabschnitts (25) anstößt, wenn der Klemmring in Richtung auf das zweite Ende (9b) zubewegt wird.

3. Die Knochenverankerungsvorrichtung gemäß Anspruch 1 oder 2, wobei der erste Eingriffsabschnitt (85) dazu eingerichtet ist, sich flexibel in Bezug auf den zweiten Eingriffsabschnitt (25) zu bewegen.

4. Die polyaxiale Knochenverankerungsvorrichtung gemäß einem der Ansprüche 1 bis 3, wobei der Stabaufnahmeabschnitt (9) wenigstens einen Ausschnitt (24) umfasst, welcher sich an dem einen Ende des Kanals von dem Boden bis zu dem zweiten Ende (9b) erstreckt, und wobei der Klemmring wenigstens einen Vorsprung (81, 83) besitzt, welcher sich durch den Ausschnitt (24) in den Kanal (12) erstreckt, wenn sich der Klemmring in einer Position befindet, in welcher der Kopf nicht geklemmt ist.

5. Die Knochenverankerungsvorrichtung gemäß Anspruch 4, wobei der Vorsprung (81) und der Federabschnitt (81, 83) voneinander getrennt sind, und der Federabschnitt (83) flexibel in Richtung auf den Vorsprung (81) bewegt werden kann.

6. Die Knochenverankerungsvorrichtung gemäß einem der Ansprüche 1 bis 5, wobei das freie Ende (83a) des Federabschnitts (83) einen Halt für den Stab (6) bereitstellt.

7. Die Knochenverankerungsvorrichtung gemäß einem der Ansprüche 4 bis 6, wobei Ausschnitte (24) an den beiden Enden des Kanals vorgesehen sind, und zwei diametral gegenüberliegende Vorsprünge (81) an dem Klemmring vorgesehen sind, die sich durch die Ausschnitte (24) in den Kanal erstrecken, und wobei entsprechend jedem Vorsprung (81) jeweils zumindest ein Eingriffsabschnitt (85) vorgesehen ist.

8. Die Knochenverankerungsvorrichtung gemäß einem der Ansprüche 1 bis 7, wobei der Klemmring (8) eine geöffnete Position einnehmen kann, in welcher der Kopfaufnahmeabschnitt (16) nicht zusammengedrückt ist, um das Einführen des Kopfs (3) zu ermöglichen, und wobei vorzugsweise in der geöffneten Position sich der erste Eingriffsabschnitt (85) in den Kanal (12) erstreckt.

9. Die Knochenverankerungsvorrichtung gemäß einem der Ansprüche 1 bis 8, wobei der Klemmring (8) eine Klemmposition einnehmen kann, in welcher er den Kopfaufnahmeabschnitt (16) zusammendrückt, um den Kopf zu klemmen.

10. Die Knochenverankerungsvorrichtung gemäß Anspruch 9, wobei sich der erste Eingriffsabschnitt (85) in der Klemmposition in den Ausschnitt (24) hinein erstreckt, und nicht im Eingriff mit dem zweiten Eingriffsabschnitt (25) steht.

11. Die Knochenverankerungsvorrichtung gemäß einem der Ansprüche 1 bis 10, wobei sich der erste und der zweite Eingriffsabschnitt im Wesentlichen in einer Kreisumfangsrichtung des Klemmrings (8) erstrecken.

12. Die Knochenverankerungsvorrichtung gemäß einem der Ansprüche 1 bis 11, wobei der Durchmesser des Kopfaufnahmeabschnitts (16) des Aufnahmeteils (5) benachbart zu dem zweiten Ende kleiner ist als der Durchmesser des Stabaufnahmeabschnitts (9) an dem zweiten Ende (9b), und/oder wobei der Außendurchmesser des Klemmrings (8) kleiner oder gleich dem Außendurchmesser der Stabaufnahmeabschnitts (9) ist.

13. Die Knochenverankerungsvorrichtung gemäß einem der Ansprüche 1 bis 12, wobei der Kopfaufnahmeabschnitt (16) eine Vielzahl von Schlitzen (20) umfasst, die sich zu dem freien Ende (17) hin öffnen, und/oder wobei der Stabaufnahmeabschnitt (9) eine Vielzahl von Schlitzen (15) in seiner Wand umfasst.

14. Die Knochenverankerungsvorrichtung gemäß einem der Ansprüche 1 bis 13, wobei der Kopf (3) sphärisch-segmentförmig ist, und der zweite Abschnitt eine innere Oberfläche mit einem entsprechend sphärischen Abschnitt (18) umfasst, um eine Schwenkbewegung des Kopfs (3) zu ermöglichen.

15. Die Knochenverankerungsvorrichtung gemäß einem der Ansprüche 1 bis 14, wobei ein Verschlusselement (7), vorzugsweise eine Setzschraube, zum Sichern des Stabs in der Ausnehmung vorgesehen ist.

## Revendications

1. Dispositif d'ancrage osseux comprenant
un élément d'ancrage osseux (1) ayant un arbre (2) pour l'ancrage dans l'os et une tête (3) ;
une partie de réception (5) pour coupler une tige (6) à l'élément d'ancrage osseux, où la partie de réception comprend
une partie de réception de tige (9) avec une première extrémité (9a) et une deuxième extrémité (9b) et un canal (12) avec un fond destiné à recevoir la tige, et
une partie de réception de tête (16) au côté de la deuxième extrémité (9b) pour recevoir la tête (3), la partie de réception de tête ayant une extrémité libre (17) et étant flexible de manière à permettre l'introduction et le serrage de la tête ;
un anneau de verrouillage (8) entourant la partie de réception de tête (16) ;
où la tête (3) est verrouillée par compression de la partie de réception de tête (16) avec l'anneau de verrouillage ;
où une première partie d'engagement (85) est prévue au niveau de l'anneau de verrouillage et une deuxième partie d'engagement (25) est prévue au niveau de la paroi interne du canal (12) ;
**caractérisé en ce que**
la première partie d'engagement (85) est prévue sur une partie de ressort (83) qui s'étend sur le canal (12) et que la première partie d'engagement (85) s'engage avec la deuxième partie d'engagement (25) pour maintenir l'anneau de verrouillage (8) dans une position où la tête (3) peut être pivotée dans la partie de réception de tête et est empêchée d'en être retirée.

2. Dispositif d'ancrage osseux de la revendication 1, dans lequel la première partie d'engagement (85) présente une surface (85a) qui vient en butée contre une surface (25a) de la deuxième partie d'engagement (25) lorsque l'anneau de verrouillage se déplace vers la deuxième extrémité (9b).

3. Dispositif d'ancrage osseux de la revendication 1 ou 2, dans lequel la première partie d'engagement (85) est configurée de sorte à se déplacer de manière flexible par rapport à la deuxième partie d'engagement (25).

4. Dispositif d'ancrage osseux polyaxial selon l'une des revendications 1 à 3, dans lequel la partie de réception de tige (9) comprend au moins une partie ajourée (24) qui s'étend au niveau d'une extrémité du canal depuis le fond vers la deuxième extrémité (9b) et où l'anneau de verrouillage comporte au moins une saillie (81, 83) qui s'étend à travers la partie ajourée (24) dans le canal (12) lorsque l'anneau de verrouillage est dans une position où la tête n'est pas verrouillée.

5. Dispositif d'ancrage osseux de la revendication 4, dans lequel la saillie (81) et la partie de ressort (81, 83) sont séparées et la partie de ressort (83) peut se déplacer de manière flexible vers la saillie (81).

6. Dispositif d'ancrage osseux de l'une des revendications 1 à 5, dans lequel l'extrémité libre (83a) de la partie de ressort (83) fournit un support pour la tige (6).

7. Dispositif d'ancrage osseux de l'une des revendications 4 à 6, dans lequel des parties ajourées (24) sont prévues à chaque extrémité du canal et deux saillies diamétralement opposées (81) sont prévues au niveau de l'anneau de verrouillage s'étendant à travers les parties ajourées (24) dans le canal et où au moins une partie d'engagement (85) correspondant à chaque saillie (81) est prévue.

8. Dispositif d'ancrage osseux de l'une des revendications 1 à 7, dans lequel l'anneau de verrouillage (8) peut adopter une position ouverte dans laquelle la partie de réception de tête (16) n'est pas comprimée pour permettre d'introduire la tête (3), et dans lequel de préférence dans la position ouverte la première partie d'engagement (85) s'étend dans le canal (12).

9. Dispositif d'ancrage osseux de l'une des revendications 1 à 8, dans lequel l'anneau de verrouillage (8) peut adopter une position de verrouillage dans laquelle il comprime la partie de réception de tête (16) pour verrouiller la tête.

10. Dispositif d'ancrage osseux de la revendication 9, dans lequel dans la position de verrouillage la première partie d'engagement (85) s'étend dans la partie ajourée (24) et est hors de l'engagement avec la deuxième partie d'engagement (25).

11. Dispositif d'ancrage osseux de l'une des revendications 1 à 10, dans lequel les première et deuxième parties d'engagement s'étendent essentiellement dans une direction circonférentielle de l'anneau de verrouillage (8).

12. Dispositif d'ancrage osseux selon l'une des revendications 1 à 11, dans lequel le diamètre de la partie de réception de tête (16) de la partie de réception (5) adjacente à la deuxième extrémité est plus petit que le diamètre de la partie de réception de tige (9) au niveau de la deuxième extrémité (9b), et/ou dans lequel le diamètre externe de l'anneau de verrouillage (8) est inférieur ou égal au diamètre externe de la partie de réception de tige (9).

13. Dispositif d'ancrage osseux selon l'une des revendications 1 à 12, dans lequel la partie de réception de tête (16) comprend plusieurs fentes (20) s'ouvrant vers l'extrémité libre (17) et/ou dans lequel la partie de réception de tige (9) comprend plusieurs fentes (15) dans sa paroi.

14. Dispositif d'ancrage osseux selon l'une des revendications 1 à 13, dans lequel la tête (3) est en forme de segment sphérique et la deuxième partie comprend une surface interne avec une partie sphérique correspondante (18) pour permettre un mouvement de pivotement de la tête (3).

15. Dispositif d'ancrage osseux selon l'une des revendications 1 à 14, dans lequel un élément de fermeture (7), de préférence une vis de serrage, est prévu pour fixer la tige dans l'évidement.
